# EUROPEAN PATENT SPECIFICATION

(11) **EP 4 493 623 B1**
(45) Date of publication and mention of the grant of the patent: **21.01.2026**
(21) Application number: 23713764.1
(22) Date of filing: 15.03.2023
(51) Int. Cl.: C08J 11/24, B01J 31/02, C07C 67/03, C07C 69/82

(54) **PET DEPOLYMERIZATION PROCESS**
PET-DEPOLYMERISATIONSVERFAHREN
PROCÉDÉ DE DÉPOLYMÉRISATION DE PET

(30) Priority: 16.03.2022 IT 202200005180
(43) Date of publication of application: 22.01.2025
(73) Proprietor: Repet S.r.l., 00142 Roma (IT)
(72) Inventor: CONTE, Davide, 00198 Roma (IT); DI TRAPANI, Simone, 00198 Roma (IT)
(74) Representative: Studio Torta S.p.A.
(86) International application number: PCT/IB2023/052504
(87) International publication number: WO 2023/175524

(56) References cited:
- CN-A- 108 484 392
- CN-A- 111 217 700
- CN-A- 112 851 502
- XUE-DAN HOU ET AL: "Effect of anion structures on cholinium ionic liquids pretreatment of rice straw and the subsequent enzymatic hydrolysis", BIOTECHNOLOGY AND BIOENGINEERING, JOHN WILEY, HOBOKEN, USA, vol. 112, no. 1, 10 September 2014 (2014-09-10), pages 65 - 73, XP071146000, ISSN: 0006-3592, DOI: 10.1002/BIT.25335
- RADOSEVIC KRISTINA ET AL: "Comparativein vitrostudy of cholinium-based ionic liquids and deep eutectic solvents toward fish cell line", ECOTOXICOLOGY AND ENVIRONMENTAL SAFETY, ACADEMIC PRESS, SAN DIEGO, CA, US, vol. 131, 13 May 2016 (2016-05-13), pages 30 - 36, XP029564902, ISSN: 0147-6513, DOI: 10.1016/J.ECOENV.2016.05.005
- CVJETKO BUBALO MARINA ET AL: "Cholinium-based deep eutectic solvents and ionic liquids for lipase-catalyzed synthesis of butyl acetate", JOURNAL OF MOLECULAR CATALYSIS B : ENZYMATIC, vol. 122, 12 September 2015 (2015-09-12), pages 188 - 198, XP029314714, ISSN: 1381-1177, Retrieved from the Internet <URL:https://www.sciencedirect.com/science/article/pii/S1381117715300667?via%3Dihub> [retrieved on 20221025], DOI: 10.1016/J.MOLCATB.2015.09.005
- LIU YACHAN ET AL: "Degradation of poly(ethylene terephthalate) catalyzed by metal-free choline-based ionic liquids", GREEN CHEMISTRY, vol. 22, no. 10, 26 May 2020 (2020-05-26), GB, pages 3122 - 3131, XP055974112, ISSN: 1463-9262, DOI: 10.1039/D0GC00327A
- MARULLO SALVATORE ET AL: "Amino Acid-Based Cholinium Ionic Liquids as Sustainable Catalysts for PET Depolymerization", vol. 9, no. 45, 15 November 2021 (2021-11-15), US, pages 15157 - 15165, XP055973834, ISSN: 2168-0485, Retrieved from the Internet <URL:https://pubs.acs.org/doi/pdf/10.1021/acssuschemeng.1c04060> DOI: 10.1021/acssuschemeng.1c04060

## Description

### FIELD OF THE ART

The present invention relates to a PET depolymerization process and the preparation of a specific ionic liquid necessary for the depolymerization process.

### BACKGROUND

Polyethylene terephthalate (PET) is one of the most widely used polymeric materials due to its characteristics in terms of electrical properties, chemical resistance, performance at high temperatures, and moulding speed. In this respect, PET has long been the main material used in the food packaging industry. In fact, PET packaging is now also commonly used, in addition to bottles, for producing trays. In particular, features that contribute to making PET an extremely suitable material for food packaging include an efficient recycling loop in almost all continents and high material availability from the recycling loop.

In this respect, polyethylene terephthalate may be disposed of either by chemical recycling or by mechanical recycling. Chemical recycling consists in the depolymerization of the product powder by converting polyethylene terephthalate into the initial raw material.

While chemical recycling allows PET powder to be returned to its monomer state to be subsequently reused for producing PET also for food use, mechanical recycling produces lower quality products of but at the same time is far cheaper. For example, products resulting from mechanical recycling may not be used for the food industry, except for mineral water and soft drinks containers according to special modes.

One way of carrying out chemical recycling relates to depolymerization by glycolysis. Glycolysis is a type of chemical recycling method which converts waste PET into monomer through various chemical reactions. Compared to other recycling methods, the most obvious advantage of glycolysis is that the monomer may be re-polymerized into new PET materials under relatively mild reaction and low-volatile solvent conditions.

In recent years, PET depolymerization processes by glycolysis have been implemented using ionic liquids (ILs) as catalysts. Ionic liquids are organic salts with a low melting point and are considered a safer alternative than traditional organic solvents due to their low vapour pressure and non-flammability. Naturally occurring aliphatic cation ionic liquids, such as (2-hydroxyethyl)trimethylammonium, also known as cholinium, have drawn great interest.

To date, the processes developed for depolymerizing PET using ionic choline liquids provide pressure and temperature conditions such as to make them unattractive when applied on an industrial scale.

The need was therefore felt to make available a process for depolymerizing PET with choline ionic liquid, the technical characteristics of which were such as to make it more industrially attractive than the processes of the prior art.

Patent Document CN 112 851 502 A discloses a method for catalyzing the methanol alcoholysis of waste PET polyester using a choline based ionic liquid, wherein the choline based ionic liquid includes choline glycine [Cho] [Gly], choline proline [Cho] [Pro], choline formic acid, choline acetic acid, choline propionic acid, or choline butyric acid.

The article by Liu, Y., entitled "Degradation of poly(ethylene terephthalate) catalyzed by metal-free choline-based ionic liquids", Green Chemistry, vol. vol. 22, no. 10, 26-05-2020, pages 3122-3131, describes a method for depolymerizing poly(ethylene terephthalate) (PET) by glycolysis to its monomer bis(hydroxyethyl) terephthalate (BHET) using choline-based ionic liquids: namely choline formate ([Ch] [For]), choline acetate ([Ch] [OAc]), choline propionate ([Ch] [Pro]), choline butyrate ([Ch][But]), choline mesylate ([Ch][Mesy]), and choline bisulfate ([Ch][HSO4]).

The article by Marullo S., entitled "Amino Acid-Based Cholinium Ionic Liquids as Sustainable Catalysts for PET Depolymerization", ACS Sustainable Chemistry & Engineering, vol. 9, no. 45, 15-11-2021, pages 15157-15165, describes a method for depolymerizing poly(ethylene terephthalate) (PET) by glycolysis in the presence of cholinium glycinate ([Ch] [Gly]), cholinium lysinate ([Ch] [Lys]), cholinium alaninate ([Ch][Ala]).

Patent Document CN 111 217 700 A discloses a method for catalyzing the alcoholysis of polyethylene terephthalate (PET) by non-metallic choline ionic liquid, wherein the cation is choline and the anion is [HCOO]⁻ or [CH₃COO]⁻ or [C₂H₅COO]⁻ or [C₃H₇COO]⁻.

Patent Document CN 108 484 392 A discloses an eutectic ionic liquid catalyst for the preparation of diisooctyl terephthalate (DOTP) by alcoholysis of polyethylene terephthalate (PET), wherein the ionic liquid catalyst is prepared by using choline chloride and metal salts: zinc acetate, zinc chloride, zinc nitrate, zinc sulfate, manganese acetate and copper nitrate.

### OBJECT AND SUMMARY OF THE INVENTION

The inventors of the present invention have implemented a process capable of satisfying the above need.

Two and a half years ago, the inventors devised the PET depolymerization process according to the present invention. At that time, choline-based ionic liquids were beginning to take their first steps by means of publications that focused mainly on their chemical-physical characterisation, which was still lacking in possible applications.

It was only in the spring of 2020 that the first paper was published suggesting the possibility of applying choline-based ionic liquids as catalysts in PET depolymerization processes by means of glycolysis (in which, furthermore, the catalytic function has not been clearly demonstrated). To date, all the publications related to the use of choline-based ionic liquids as catalysts in PET glycolytic depolymerization show no particular developments.

Conversely, during the period from August 2019 to March 2021, the inventors tested their technology firstly with multiple computational simulations (until December 2020) and then through laboratory experiments (until August 2021).

The object of the present invention is a PET depolymerization process, the essential features of which are reported in the independent claim 1, and the secondary and auxiliary features of which are reported in the dependent claims 2 - 6.

### DETAILED DESCRIPTION OF PREFERRED EMBODIMENTS OF THE INVENTION

Embodiments are hereinafter shown for merely illustrative and non-limiting purposes.

### Choline argininate (Cho-l-Arg)ionic liquid synthesis

0.70 grams of l-arginine powder at the highest available purity were introduced into a 20 ml vial and a suitably sized magnetic stirrer.

1 ml of an aqueous solution of 2-hydroxy-N,N-trimethylethanammonium (ChoOH)was added to the vial drop by drop. The amount of added ChoOH represents a number of moles equal to that of l-arginine. In addition, 7 ml of MilliQ water were also added to the vial. At this point, a constant 350 rpm rotation was set on the magnetic stirrer, while the vial was maintained at a temperature of 30°C by means of a thermostat bath. These conditions were maintained for a time interval of 48h.

Adding the ChoOH aqueous solution directly to the l-Arginine powder is necessary to prevent the acid-base equilibrium of the guanidine group of the amino acid, which would make the metathesis reaction between the acid proton of the carboxylic group of the amino acid and the hydroxyl group of the choline hydroxide more difficult.

It was verified that if this addition is carried out drop by drop or in a single solution, on an aqueous solution of l-arginine, maintaining an equal number of moles between l-arginine and choline hydroxide in both solutions, the Cho-l-Arg yield decreased significantly from 100% to about 30%. Subsequently, the contents of the vial were transferred to a 50 mL volume flask.

At this point, the water was removed by means of a rotavapor (pressure values of approx. 35-30 mbars), keeping the water bath first at 30°C and then, after approx. 40% water removal, at 40°C. The inventors believe that the step of removing water from the reaction matrix must be a time-consuming process which requires great care. From the above, a compound, exceeding from zero to 3.5% by mass, was obtained.

### PET depolymerization

An example of depolymerization carried out in the laboratory is hereinafter reported.

As may be immediately apparent to a person skilled in the art, the example reported below aims to demonstrate the depolymerization effectiveness of (2-hydroxyethyl) trimethylammonium argininate according to the process of the invention. In this respect, the reported yields are not to be regarded as particularly significant as they may be improved by a cyclic repetition of the process or by industrial adjustments falling outside the characteristics defined in the claims.

9.9936 g of PET from a transparent bottle and cut into pieces of approximately 1x0.5 cm in size and 1.4108 g of ionic liquid consisting of (2-hydroxyethyl)trimethylammonium argininate ([Cho] [Arg]) prepared as described above were introduced into a 50 ml reaction flask.

The reaction flask was fixed to a rotavapor with a thermostat bath at 60°C to allow the viscosity of the ionic liquid to be reduced and the contact with the PET to be increased accordingly. Under these conditions, the rotavapor was activated at a speed of 90 rpm and kept running for 90 minutes.

After 90 minutes, the temperature of the thermostat bath was lowered to 30°C and the rotavapor was kept in operation for a further 120 minutes.

After the 120 minutes had elapsed, the rotation was stopped and approximately 2 ml of methanol were added drop by drop. Once the addition of methanol was completed, the rotation was reactivated under the same conditions as described above (90 rpm and 30°C). After 30 minutes of rotation only (without adding methanol) the rotation was stopped and another 2 ml of methanol were added, drop by drop. Once the addition of methanol was completed, the rotation under the above conditions was reactivated for a further 30 minutes.

This operation was repeated 10 times.

According to a further embodiment, an alkali metal methoxide may also be added together with methanol.

Once the methanol additions were completed, the rotation itself was stopped and the non-depolymerized PET was recovered via a buchner funnel and washed with methanol under moderate stirring for 10 minutes. The washing methanol was then recovered and added to the depolymerization matrix consisting of a white precipitate, [Cho][Arg] and methanol.

The non-depolymerized PET was subsequently washed, to be suitably dried and weighed. It was thereby proved that non-depolymerized PET weighed 9.0105 g. A depolymerization yield of about 10% may be inferred from the above.

20 ml of MilliQ water were added to the depolymerization matrix and rotation at 90 rpm was reactivated until the white precipitate was completely dissolved. At this point, the contents of the reaction flask were poured into a beaker together with an additional 150 ml of MilliQ water and a stir bar. The beaker was housed in a crystalliser equipped with an ice bath and the beaker was rotated at a speed of 500 rpm for 15 minutes.

Subsequently, a 1M of HCl solution was added drop by drop to the beaker until the solution in the beaker reached pH 1. Under these conditions, the formation of a white precipitate was noted.

At this point, filtration was performed with a funnel and blue filter paper and the white precipitate was washed thoroughly with more MilliQ water. Once recovered, the white precipitate was dried by placing it in a crystalliser in an oven at a temperature of 94°C for 1h.

Once dried, the white compound was submitted to 1h-nmr, ATR, uv-vis spectrophotometric analysis. As shown by the enclosed spectra, spectrophotometric analysis confirmed that the white compound is terephthalic acid.

The inventors considered that the above reported procedure may be effective at any temperature that keeps the reagents in their liquid state. For this reason, it was considered that in the temperature range from -50 to 70 °C, the above reported depolymerization procedure can take place.

In case depolymerization is carried out at a higher temperature than indicated, a number of problems may be involved such as degradation of the (2-hydroxyethyl) trimethylammonium argininate, solubilisation of the monomer reaction product (DMT) within the reaction matrix and subsequent slowing down of the depolymerization process and evaporation of the solvent (CH3OH).

Furthermore, as may be obvious to a person skilled in the art, the use of a higher temperature than indicated necessarily leads to productivity problems if the process of the invention is applied on an industrial scale.

The inventors of the present invention considered the optimal temperature range to be between 20 and 40°C.

Furthermore, the inventors of the present invention believe that the ratio of PET to ionic liquid may be unbalanced with respect to both PET and ionic liquid without affecting the effectiveness of depolymerisation.

### COMPARATIVE EXAMPLES

For comparative purposes, three comparative examples were made in which [Cho][Arg] was replaced with [Cho][Gly], [Cho][Pro] and L-Arginine, respectively.

### - Example with [Cho][Gly]

In this example, 1.0830 g of [Cho][Gly] and 1.0980 g of PET were used

The time and temperature conditions of the depolymerization example according to the above invention were repeated, while the amount of methanol added, drop by drop, each time is of 0.1 ml.

[Cho][Gly] was synthesised in the laboratory using the same synthesis procedure as in the case of choline argininate and described above. The reagents used are all Merck-certified.

At the end of the depolymerisation procedure, the recovered non-depolymerized PET was found to weigh 1.0989 g and thus 0.082 % more (within the weighing error of the balance) than the PET initially used.

It is clear from the above that the use of the ionic liquid consisting of [Cho][Gly] does not produce any depolymerization.

### - Example with [Cho][Pro]

In this example, 1.1620 g of [Cho][Pro] and 1.0452 g of PET were used

The time and temperature conditions of the depolymerization example according to the above invention were repeated, while the amount of methanol added, drop by drop, each time is of 0.1 ml.

[Cho] [Pro] was synthesised in laboratory using the same synthesis procedure as in the case of choline argininate and described above. The reagents used are all Merck-certified.

At the end of the depolymerisation procedure, the recovered non-depolymerized PET was found to weigh 1.0511 g and thus 0.0560 % more (within the weighing error of the balance) than the PET initially used.

It is clear from the above that the use of the ionic liquid consisting of [Cho][Pro] does not produce any depolymerization.

### - Example with L-Arginine

In this example, 0.7084 g L-Arginine and 1.1021 g PET were used.

The time and temperature conditions of the depolymerization example according to the above invention were repeated, while the amount of methanol added, drop by drop, each time is of 0.1 ml.

At the end of the depolymerization procedure, the recovered non-depolymerized PET weighed 1.1022 g.

It is clear from the above that the use of L-Arginine (Merck-certified) does not produce any depolymerization.

From the above description, it appears that, surprisingly, only the use of ionic liquid [Cho] [Arg] is able to obtain PET depolymerization under ambient temperature and pressure conditions. Such a result offers important production and environmental advantages. In fact, the process of the present invention allows PET to be depolymerized to obtain the starting monomer, which can, in turn, be polymerized again.

It is important to note that the process of the present invention, when applied to materials comprising PET, succeeds in extracting PET by depolymerization, leaving the other constituent components of the material available.

In this regard, an example of depolymerization of textile fibre carried out in the laboratory is hereinafter reported.

### - Example of textile fibre depolymerization -

A 50 ml volume flask was loaded with a textile fibre sample consisting of a flap of fabric weighing 0.7158 g (composed of 65 % polyester and 35 % cotton) and 0.6260 g of ionic liquid consisting of (2-hydroxyethyl)trimethylammonium argininate ([Cho][Arg]). After a blending step carried out at 30 °C between the ionic liquid and the textile fibre using the rotor of a rotavapor, 1 ml of methanol was added over the following nine hours (0.1 ml every hour). Rotation was stopped after each addition to allow methanol to be introduced into the reaction matrix. Subsequently, a further 2.4 ml of methanol was introduced by directly loading 0.4 ml six times. The rotor was temporary interrupted at each addition.

The depolymerization reaction was then interrupted by adding MilliQ water (approximately 50 ml) to the reaction matrix. The residual sample was washed in a beaker with double-distilled water and dried, yielding a weight of 0.5331 g.

The reaction matrix was then filtered by removing cotton polymer residues. The PET monomer was then extracted by additions of 1 M of HCl until pH 1 was reached. The terephthalic acid was collected by filtration, dried in an electric oven and weighed, yielding a mass of 0.14766 g compared to an initial polyester mass of 0.46527 g.

It may therefore be estimated a depolymerization on the PET textile fibre of about 33%.

## Claims

1. A PET depolymerization process obtained by using a ionic liquid comprising choline; said depolymerisation process being **characterized in that** PET, a ionic liquid consisting of (2-hydroxyethyl) trimethylammonium argininate [Cho][Arg] and methanol are mixed together at a temperature ranging from -50 to 70°C.

2. The process according claim 1, **characterized in that** the weight ratio between PET and the ionic liquid ranges from 10 to 0.05.

3. The process according to claim 1 or 2, **characterized in that** it comprises (a) a first mixing step, during which PET is mixed with the ionic liquid consisting of (2-hydroxyethyl)trimethylammonium argininate [Cho] [Arg] at a temperature ranging from 20 to 70°C, and (b) a depolymerization step, during which the mixture obtained from said mixing step is added to ROH alcohol and stirred at a temperature ranging from -50 to 70°C.

4. The process according to one of the preceding claims, **characterized in that** methoxide of an alkali metal is added together with methanol.

5. The process according to one of the preceding claims, **characterized in that** said methanol is added to the mixture drop by drop.

6. The process according to one of the preceding claims, **characterized in that** said depolymerization step comprises the repetition of a methanol adding operation and of a mixing operation one after the other.

## Patentansprüche

1. PET-Depolymerisationsverfahren, das unter Verwendung einer ionischen Flüssigkeit erhalten wird, die Cholin umfasst; wobei das Depolymerisationsverfahren **dadurch gekennzeichnet ist, dass** PET, eine ionische Flüssigkeit, die aus (2-Hydroxyethyl)trimethylammoniumargininat [Cho] [Arg] und Methanol besteht, bei einer Temperatur im Bereich von -50 bis 70 °C miteinander vermischt werden.

2. Verfahren nach Anspruch 1, **dadurch gekennzeichnet, dass** das Gewichtsverhältnis zwischen PET und der ionischen Flüssigkeit im Bereich von 10 bis 0,05 liegt.

3. Verfahren nach Anspruch 1 oder 2, **dadurch gekennzeichnet, dass** es (a) einen ersten Mischschritt, bei dem PET mit der ionischen Flüssigkeit, die aus (2-Hydroxyethyl)trimethylammoniumargininat [Cho] [Arg] besteht, bei einer Temperatur im Bereich von 20 bis 70 °C gemischt wird, und (b) einen Depolymerisationsschritt, bei dem die aus dem Mischschritt erhaltene Mischung zu ROH-Alkohol gegeben und bei einer Temperatur im Bereich von -50 bis 70 °C gerührt wird, umfasst.

4. Verfahren nach einem der vorhergehenden Ansprüche, **dadurch gekennzeichnet, dass** Methoxid eines Alkalimetalls zusammen mit Methanol zugegeben wird.

5. Verfahren nach einem der vorhergehenden Ansprüche, **dadurch gekennzeichnet, dass** das Methanol der Mischung tropfenweise zugesetzt wird.

6. Verfahren nach einem der vorhergehenden Ansprüche, **dadurch gekennzeichnet, dass** der Depolymerisationsschritt die Wiederholung eines Methanolzugabevorgangs und eines Mischvorgangs nacheinander umfasst.

## Revendications

1. Procédé de dépolymérisation de PET obtenu en utilisant un liquide ionique comprenant de la choline ; ledit procédé de dépolymérisation étant **caractérisé en ce que** le PET, un liquide ionique constitué d'argininate de (2-hydroxyéthyl)triméthylammonium [Cho] [Arg] et du méthanol sont mélangés à une température comprise entre -50 et 70°C.

2. Procédé selon la revendication 1, **caractérisé en ce que** le rapport pondéral entre le PET et le liquide ionique est compris entre 10 et 0,05.

3. Procédé selon la revendication 1 ou 2, **caractérisé en ce qu'**il comprend (a) une première étape de mélange, au cours de laquelle le PET est mélangé avec le liquide ionique constitué d'argininate de (2-hydroxyéthyl)triméthylammonium [Cho] [Arg] à une température comprise entre 20 et 70°C, et (b) une étape de dépolymérisation, au cours de laquelle le mélange obtenu lors de ladite étape de mélange est ajouté à de l'alcool ROH et agité à une température comprise entre -50 et 70°C.

4. Procédé selon l'une des revendications précédentes, **caractérisé en ce que** le méthoxyde d'un métal alcalin est ajouté au méthanol.

5. Procédé selon l'une des revendications précédentes, **caractérisé en ce que** ledit méthanol est ajouté au mélange goutte à goutte.

6. Procédé selon l'une des revendications précédentes, **caractérisé en ce que** ladite étape de dépolymérisation comprend la répétition d'une opération d'ajout de méthanol et d'une opération de mélange l'une après l'autre.
